# EUROPÄISCHE PATENTSCHRIFT

(11) **EP 0 639 954 B1**
(45) Veröffentlichungstag und Bekanntmachung des Hinweises auf die Patenterteilung: **16.06.1999**
(21) Anmeldenummer: 94911137.1
(22) Anmeldetag: 09.03.1994
(51) Int. Cl.: A61B 17/22

(54) **MEDIZINISCHES INSTRUMENT FÜR ATHEREKTOMIE**
MEDICAL INSTRUMENT FOR ATHERECTOMY
INSTRUMENT MEDICAL D'ATHERECTOMIE

(30) Priorität: 11.03.1993 DE 4307642
(43) Veröffentlichungstag der Anmeldung: 01.03.1995
(60) Teilanmeldung: 98122557.6
(73) Patentinhaber: REDHA, Falah, Dr., 8302 Kloten (CH)
(72) Erfinder: REDHA, Falah, Dr., 8302 Kloten (CH)
(74) Vertreter: Jeck, Anton, Dipl.-Ing.
(86) Internationale Anmeldenummer: EP9400730
(87) Internationale Veröffentlichungsnummer: WO9420033

(56) Entgegenhaltungen:
- WO-A-89/07914
- WO-A-89/09029
- WO-A-90/02523
- US-A- 5 085 659

## Beschreibung

Die Erfindung betrifft ein medizinisches Instrument nach dem Oberbegriff des Anspruches 1.

Bei herkömmlichen Instrumenten der eingangs genannten Art, wie sie z.B. in der US-PS 4765 332 beschrieben sind, ist ein etwa zylinderförmiger Grundkörper mit in Zugrichtung weisenden Schneideabsschnitten vorgesehen, die ebenfalls zylinderförmig sind. Die scharfen Kanten der Schneideabschnitte verjüngen sich von innen nach außen, was zur Verletzung auch gesunder Gefäße führen kann. Ein weiterer Nachteil, mit dem das bekannte Instrument behaftet ist, besteht darin, daß eine Anpassung an die Arterien- bzw. Venenwände nur im begrenzten Umfang möglich ist.

Schließlich ist in der WO-A-9002523 ein medizinisches Instrument gemäß dem Oberbegriff des Anspruchs 1 offenbart, bei dem die schalenförmigen Teile (Schneidkörper) mit einem Spannkörper zusammenarbeiten. Durch diesen Spannkörper ist der Abstand der Schneidekanten voneinander zwar einstellbar, die Handhabung mit dem Instrument ist jedoch umständlich.

Ausgehend vom obigen Stand der Technik liegt der Erfindung die Aufgabe zugrunde, das gattungsgemäße Instrument so weiter zu bilden, daß sein effektiver Querschnitt einfach vergrößert und den Venen angepaßt werden kann.

Die gestellt Aufgabe wird erfindungsgemäß durch die Merkmale des Anspruches 1 gelöst.

Ein solches Instrument kann in das Gefäß mittels eines Katheters eingeführt werden, und zwar bis zur vorgesehenen Stelle, die durch Kontrastmittel lokalisiert werden kann. Der Katheter und der Körper werden so angeordnet, daß die Plaque zwischen dem Körper und dem Katheter angeordnet ist. Dann wird das Zugmittel betätigt und die vorgesehene Stelle wird abgeschabt. Dieser Vorgang kann so oft wiederholt werden, bis eine glatte Innenwand vorhanden ist. Die beim Hobeln entstandenen Späne können entweder abgesaugt oder durch den Körper, der nach dem Eingriff aus der Arterieherausgezogen wird, mitgenommen werden.

Weitere zweckmäßige und vorteilhafte Ausgestaltungen der Erfindung gehen aus den Unteransprüchen hervor.

Eine zweckmäßige Ausgestaltung der Erfindung sieht vor, daß der in den Körper hineinragende Abschnitt des Grundkörpers als ein Rohrabschnitt ausgebildet ist, in dem ein Organ zur relativen Verstellung des Körpers mit Bezug auf den Schneidekörper angeordnet ist.

Dabei ist es zweckmäßig, wenn das Organ als ein Rohrstück oder Drahtstück ausgeführt ist, das von außen her betätigbar ist. Durch die Betätigung des Organs wird auf die Schneidekörper Druck ausgeübt, so daß sie den effektiven Querschnitt des Schneidekörpers entweder vergrößern oder verkleinern, und zwar abhängig davon, ob der Körper zum Benutzer hin oder von diesem weg bewegt wird.

Eine weitere zweckmäßige Ausgestaltung sieht vor, daß die Schwenkachse der Schneidekörper sich quer zur Zugrichtung des Körpers erstreckt. Dadurch wird sichergestellt, daß die Schneidekanten zum Benutzer hin weisen und die Schneidekörper ihre wirksame bzw. unwirksame Position einnehmen können. Gemäß dem Erfindungsgedanken werden die Schneidekörper als zweiarmige Hebel ausgebildet wobei die zwei Schneidekörper eine gemeinsame Schwenkachse und jeweils einen Kraft- und einen Lastarm aufweisen. Die Schneidekörper können ihrer Ruhestellung vollständig oder annähernd vollständig im Körper untergebracht werden Dabei kann der Grundkörper einen Anschlagabschnitt aufweisen, der in Ruhestellung der Schneidekörper annähernd abstandfrei zu einer Stirnseite des Körpers angeordnet ist. Der Vorteil dieser Maßnahme besteht insbesondere darin, daß das Einbringen des Intrumentes in Gefäße deutlich erleichtert wird, da in diesem Fall die Schneidekanten mit dem Gefäß nicht in Berührung kommen.

Die Innenwand des Körpers kann Druckkörper aufweisen, die in ihrer Betriebsstellung mit dem jeweiligen Kraftarm und in ihrer Ruhestellung mit dem jeweiligen Lastarm der Schneidekörper in Druckverbindung stehen. Die Druckkörper können als radiale Vorsprünge mit Gleitkurven ausgebildet sein. Der Vorteil dieser Maßnahmen besteht darin, daß durch axiale Versetzung des Körpers in Zugrichtung die Schneidekörper entweder geöffnet oder geschlossen werden. Beim Öffnen fahren die Schneidekörper aus dem Körper heraus, dabei wird auf den jeweiligen Kraftarm des Schneidekörpers seitens des Körpers Druck ausgeübt, so daß sich die Schneidekörper öffnen und außerhalb des Körpers angeordnet sind. Die Schneidekanten der Schneidekörper definieren hierbei einen Kreis oder eine Ellipse, der bzw. die außerhalb des Körpers angeordnet ist. Um die Schneidekörper kontinuierlich betätigen zu können, ist vorgesehen, daß das Außenprofil des jeweiligen Schneidekörper-Längsschnittes bogenförmig ist. Durch diese Maßnahmen wird ein allmähliches Öffnen bzw. Schließen der Schneidekörper sichergestellt.

Ferner ist vorgesehen, daß der Körper im wesentlichen als ein Hohlzylinder ausgebildet ist, der die Schenidekörper in ihrer Ruhestellung aufnimmt.

Eine weitere zweckmäßige Ausgestaltung der Erfindung sieht vor, daß die vom Benutzer abgekehrte Stirnseite der Körper eine in Zugrichtung sich erstreckende Öffnung aufweist. Die Öffnung kann zur Aufnahme eines Führungsmittels, z.B. eines dünnen Drahtstückes, dienen, durch das die Einbringung des Instrumentes an die vorgesehene Stelle erleichtert werden kann. Dabei ist es sweckmäßig, wenn die vom Benutzer abgekehrte Stirnseite des Körpers konvex und kantfrei ist.

Eine weitere zweckmäßige Ausgestaltung sieht vor, daß das als Seil-, Rohr- oder Drahtstück ausgebildete Organ Klemmkörper trägt, die abhängig von der Betriebslage der Schneidekörper mit dem Kraft- bzw. Lastarm des jeweiligen Schneidekörpers zusammenarbeiten. Dabei können die Klemmkörper im axialen Querschnitt keilförmig ausgebildet und mit ihren verjüngten Stirnseiten einander zugekehrt sein. Ferner ist es besonders zweckmäßig, wenn zwei Klemmkörper vorgesehen sind, zwischen denen die Schwenkachse der Schneidekörper angeordnet ist. Einer der Klemmkörper kann hierbei vom Grundkörper getragen sein und aus einem elastisch verformbaren Werkstoff bestehen. In diesem Fall übt nur einer der Klemmkörper Relativbewegungen mit Bezug auf die Schwenkachse der Schneidekörper aus.

Eine vorteilhafte Ausgestaltung der Erfindung sieht vor, daß die an die Schneidekanten sich anschließenden Flanken der Schneidekörper zumindest teilweise mit Schneideabschnitten versehen sind. Durch diese Maßnahmen ist sichergestellt, daß beim Schließen der Schneidekörper nicht nur die stirnseitigen Schneidekanten, sondern auch die in Längsrichtung der Schneidekörper sich ertsreckenden Kanten als Schneideorgane wirken.

Schließlich sieht eine besonders zweckmäßige Ausgestaltung der Erfindung vor, daß mehrere Schneidekörper-Paare in Serie geschaltet sind, wodurch die Beseitigung der Ablagerungen schneller erfolgen kann.

Einige Ausführungsbeispiele der Erfindung sind in der Zeichnung schematisch dargestellt und werden im folgenden näher erläutert.

Es zeigen
- Fig. 1: einen axialen Querschnitt eines Teiles der ersten Ausführungsform des Instruments in Betriebsstellung,
- Fig. 2: das in Fig. 1 dargestellete Instrument in Ruhestellung,
- Fig. 3: eine zweite Ausführungsform des Instrumentes in Betriebsstellung,
- Fig. 4: das in Fig. 3 dargestellte Instrument in Ruhestellung,
- Fig. 5: einen Schnitt entlang der Linie A-A,
- Fig. 6: eine weitere Ausführungsform des Instrumentes in seiner Betriebstellung,
- Fig. 7: eine weitere Ausführunsform des Instrumentes in seiner Schließstellung,
- Fig. 8: ein Instrument mit Druckkörpern in Betriebsstellung,
- Fig. 9: das in Fig. 8 dargestellte Instrument in Ruhestellung,
- Fig. 10: ein Instrument mit Klemmkörpern und einer Querbohrung in Betriebsstellung,
- Fig. 11: ein Instrument mit elastischen Klemmkörpern in Betriebsstellung und
- Fig. 12: das Instrument nach Fig. 1 in Tandem-Ausführung.

In den Figuren sind medizinische Instrumente zur Beseitigung von Ablagerungen in Arterien- und/oder Venenwänden mit jeweils einem in die Gefäße einbringbaren und zumindest teilweise hohlen Körper 10-14 mit einem aus den Gefäßen ausführbaren Zug- und/oder Betätigungsmittel 20 sowie mit in Zugrichtung weisenden und die Ablagerungen beseitigenden Schneidekörpern 21,22 mit Schneidekanten 24 dargestellt. Dabei ist ein in den Körper 10-14 zumindest teilweise hineinragender Grundkörper 26,27 vorgesehen, an dem die in Zugrichtung sich erstreckenden Schneidekörper 21,22 angelenkt sind und ihre Betriebs- bzw. Ruhestellung durch Verstellung des Körpers 10-14 in Zugrichtung definierbar ist. Der in den jeweiligen Körper 10-14 hineinragende Abschnitt 30 des Grundkörpers 26,27 ist als ein Rohrabschnitt ausgebildet, in dem ein Organ 32,34 zur relativen Verstellung des Körpers 10-14 mit Bezug auf den Schneidekörper 21,22 angeordnet ist. Das Organ 32,34 ist als ein Rohrstück oder Drahtstück ausgeführt, das von außen her betätigbar ist. Die Schwenkachse 36 der Schneidekörper 21,22 erstreckt sich quer zur Zugrichtung des Körpers 10-14.

Man erkennt, daß die Schneidekörper 21 und 22 als zweiarmige Hebel ausgebildet sind, die in ihren Ruhestellungen vollständig oder annähernd vollständig im Körper 10-14 untergebracht sind. Der Grundkörper 26 bzw. 27 weist einen Anschlagabschnitt 38 auf, der in Ruhestellung der Schneidekörper 21 und 22 annähernd abstandsfrei zu einer Stirnseite 40 des jeweiligen Körpers 10-14 angeordnet ist. Der jeweilige Körper 10-14 ist im wesentlichen als ein Hohlzylinder ausgebildet, der die Schneidekörper 21 und 22 in ihrer Ruhestellung aufnimmt. Die vom Benutzer abgekehrte Stirnseite 41 des jeweiligen Körpers 10-14 weist eine in Zugrichtung sich erstreckende Öffnung 42 auf, in die das Endstück des Organs 32 bzw. 34 hereinragt und dort befestigt ist. Die vom Benutzer abgekehrte Stirnseite 41 des jeweiligen Körpers 10-14 ist konvex und kantfrei.

Die Figuren lassen ferner erkennen, daß zwei Schneidekörper 21,22 mit einer gemeinsamen Schwenkachse 36 und jeweils einem Kraft- und einem Lastarm 43 und 44 vorgesehen sind. Dabei weist die Innenwand des jeweiligen Körpers 10,11,12 Druckkörper 46 auf, die in ihrer Betriebsstellung mit dem jeweiligen Kraftarm 43 und in ihrer Ruhestellung mit dem jeweiligen Lastarm 44 der Schneidekörper 21 und 22 in Druckverbindung stehen. Die Druckkörper 46 sind hierbei als radiale Vorsprünge mit Gleitkurven, die sich in axialer Richtung erstrecken, ausgebildet. Der Außenumriß des jeweiligen Schneidekörper-Längsschnittes ist bogenförmig, so daß ein kontinuierliches Öffnen bzw. Schließen der Schneidekörper gewährleistet ist.

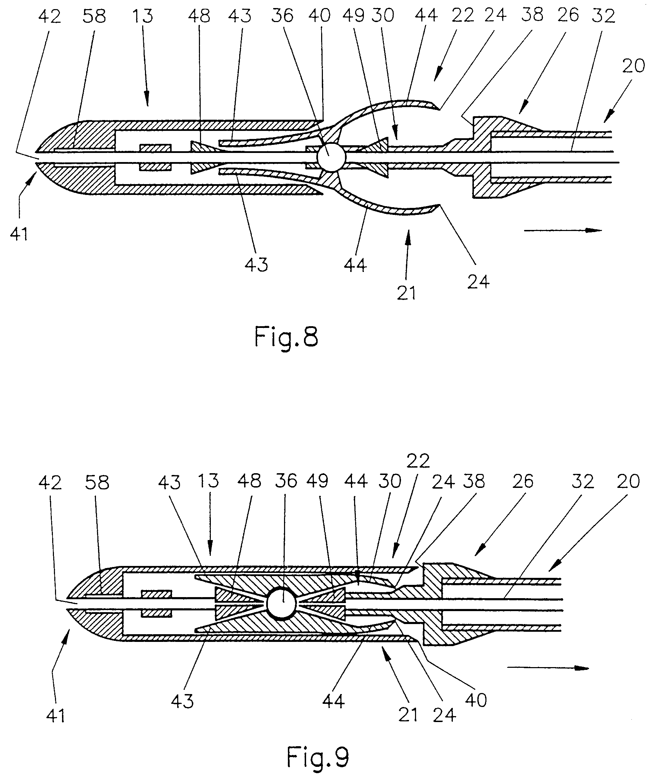

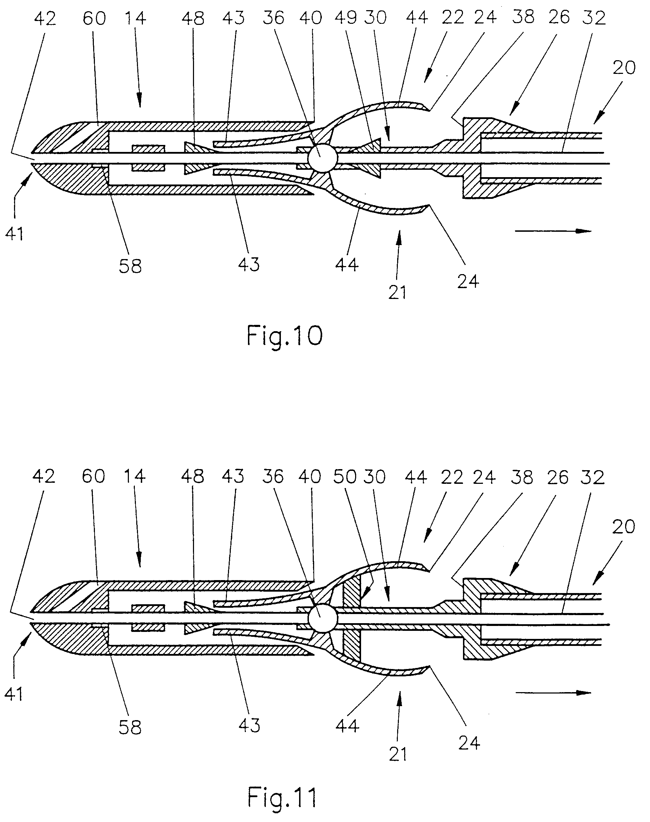

Das als Seil-, Rohr- oder Drahtstück ausgebildete Organ 32,34 trägt Klemmkörper 48,49 und 50 (vgl. Fig. 8-11), die abhängig von der Betriebslage der Schneidekörper 21 und 22 mit dem Kraft- bzw. Lastarm 43 und 44 des jeweiligen Schneidekörpers 21 und 22 zusammenarbeiten. Die Klemmkörper 48,49 und 50 sind im axialen Querschnitt keilförmig ausgebildet und mit ihren verjüngten Stirnseiten einander zugekehrt. Hierbei sind jeweils zwei Klemmkörper 48,49 und 50 vorgesehen, zwischen denen die Schwenkachse der Schneidekörper 21 und 22 angeordnet ist. Der in Fig. 11 dargestellte Klemmkörper 50 ist vom Grundkörper 26 getragen und besteht aus einem elastisch verformbaren Werkstoff.

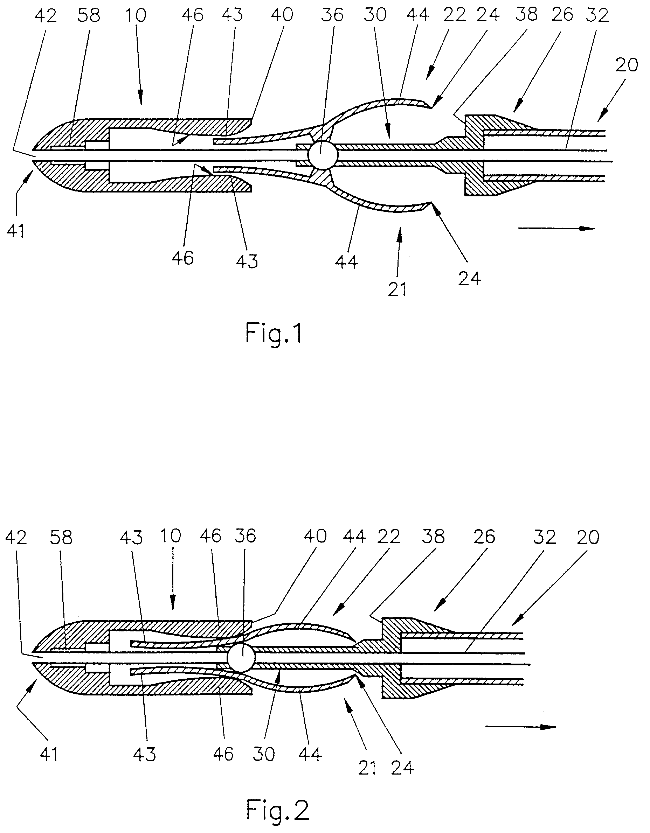

Das in Fig. 1 und 2 dargestellte Teil des Medizinischen Instruments weist ferner die Besonderheit auf, daß der Körper 10 die Schneidekörper 21 und 22 in ihrer Ruhestellung (Fig. 2) nicht vollständig aufnimmt. Die Schneidekanten 24 sind jedoch vor dem Anschlag 38 angeordnet, so daß es in diesem Fall zu keinem Schneidevorgang kommen kann. Der Schneidevorgang wird erst dann gewährleistet, wenn der Körper 10 einen etwa maximalen Abstand von der Schwenkachse 36 aufweist, wobei seine Druckkörper 46 auf die Kraftarme 43 Druck ausüben, so daß die Schneidekanten 24 maximal voneinander angeordnet sind und den Grundkörper 26 überragen, d.h. der Außenumfang des Grundkörpers 26 ist hierbei kleiner als der Außenumfang der beiden Schneidekanten 24. Da die Schneidekörper 21 und 22 am Abschnitt 30 , der als Rohrstück ausgebildet ist, angelenkt sind, wird beim Verschieben des Organs 32, das ebenfalls als ein Rohrstück ausgebildet ist, der Körper 10 wegbewegt, wobei auf die Kraftarme 43 Kraft ausgeübt wird. In das Organ 32 kann ein Führungskörper, z.B. Drahtstück, eingebracht werden, das aus der Öffnung 42 herausragt und zur Einführung des Instrumentes in die Gefäße dienen kann. Wird das Organ 32 in Richtung des Pfeiles betätigt, dann wird der Abstand des Körpers 10 von der Schwenkachse 36 minimiert, wobei die Druckkörper 46 auf die Lastarme 44 Druck ausüben, so daß das Instrument die in Fig. 2 dargestellte Position einnimmt.

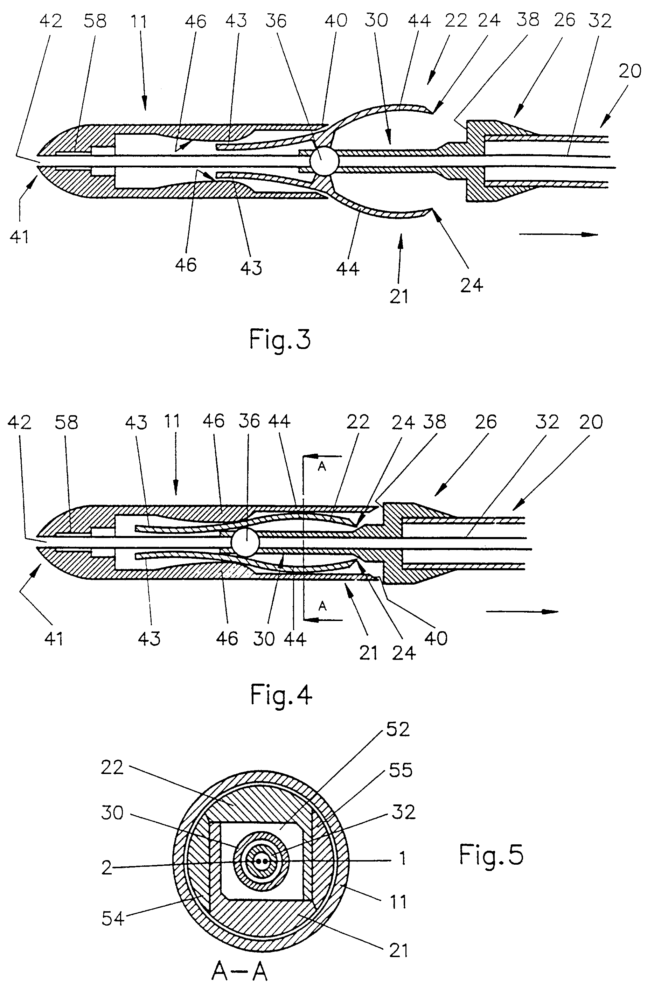

In den Figuren 3 bis 5 ist ein Teil eines weiteren Instrumentes dargestellt, das sich von dem Instrument nach Figuren 1 und 2 dadurch unterscheidet, daß der Körper 11 so bemessen ist, daß er in Schließstellung bzw. Ruhestellung des Instrumentes beide Schneidekörper 21 und 22 voll aufnehmen kann (vgl. Fig. 4). Der Außenumriß des Querschnittes des Körpers 11 ist kreisrund. Den gleichen Querschnitt hat auch der Grundkörper 26, so daß der Körper 11 mit dem Grundkörper 26 bündig abschließen kann. Ein Vorteil dieser Maßnahme besteht darin, daß das Einbringen des Instrumentes in die Gefäße besonders einfach ist.

Die Fig. 5 läßt erkennen, daß auch die in Zugrichtung des Instrumentes sich erstreckenden Flanken 54 und 55 als Schneidekörper ausgebildet sind, so daß nicht nur die Stirnseite, sondern auch die Längsseiten zum Schneiden dienen können. Das Organ 32 ist hierbei so bemessen, daß es gleichzeitig auch zur Führung eines Angioscopen 3 und eines Führungskörpers 1 dienen kann.

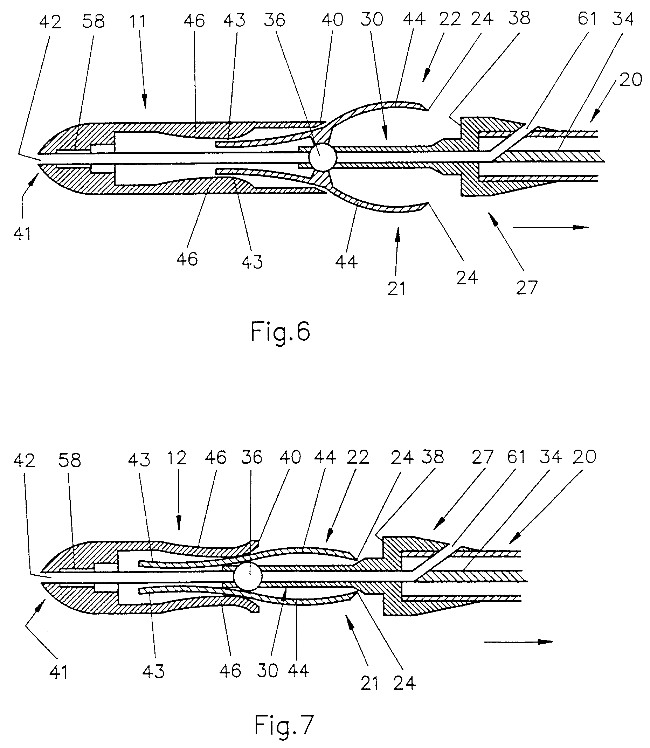

Die Besonderheit des Instrumentes, wie es in Fig. 7 dargestellt ist, besteht darin, daß der Körper 12 im mittleren Bereich eine Durchmesserverringerung aufweist, so daß hierdurch gleichzeitig ein Vorsprung für die Betätigung des Kraft- bzw. Lastarmes des jeweiligen Schneidekörpers gebildet wird. Der Grundkörper 27 besitzt eine seitliche Querbohrung 61, in die ein Führungskörper einbringbar ist, dessen freies Ende aus der Öffnung 42 herausragen kann. In diesem Fall kann das Organ 34 als ein Drahtstück ausgebildet sein.

Mit einer ähnlichen Querbohrung 61 ist auch das in Fig. 6 dargestellte Instrument versehen, wobei der Körper 11 so bemessen ist, daß er die Schneidekörper voll aufnehmen kann, wie es im Falle des Instrumentes nach Fig. 3 offenbart ist.

Die Besonderheiten der Instrumente nach den Figuren 8 bis 11 besteht zunächst darin, daß das als Seil-, Rohr-oder Drahtstück ausgebildete Organ 32 bzw. 34 die Klemmkörper 48,49 und 50 trägt, die abhängig von der Betriebslage der Schneidekörper 21 und 22 mit dem Kraft- bzw. Lastarm 43 und 44 des jeweiligen Schneidekörpers 21 und 22 zusammenarbeiten. Durch die relative Verstellung des Organs 32 mit Bezug auf den Grundkörper 26 werden auch die Klemmkörper 48 und 49 mit Bezug auf die Schwenkachse 36 verstellt, wobei sie auf den jeweiligen Last- bzw. Kraftarm innenseitig Druck ausüben, so daß diese nach innen bzw. außen verschwenkt werden. Im Falle des Instrumentes nach Fig. 10 ist das Organ 32 ein Drahtstück. Um auch in diesem Falle den Körper 14 in das Gefäß problemlos einführen zu können, ist im stirnseitigen Endstück des Körpers 14 eine Querbohrung 60 ausgebildet, in die ein Führungsdrahtstück einbringbar ist.

Die Besonderheit des Instrumentes nach Fig. 11 besteht darin, daß die Klemmkörper 50 mit dem Abschnitt 30 fest verbunden sind und aus einem elastisch verformbaren Werkstoff bestehen. Daher ist es erforderlich, nur den Klemmkörper 48 zu versetzen. Ist der Klemmkörper 48 mit den Kraftarmen 43 nicht im Eingriff, dann üben die Klemmkörper 55 auf die Lastarme 44 Kraft aus, so daß sie ausgeschwenkt werden und die in Fig. 11 dargestellte Position einnehmen.

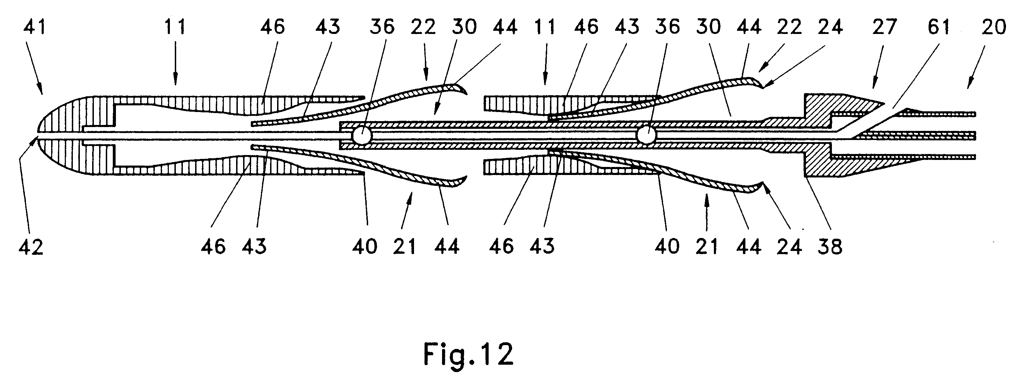

Der in Fig. 12 dargestellte Teil eines medizinischen Instrumentes zeichnet sich dadurch aus, daß zwei Schneidekörper-Paare 21,22 in Serie angeordnet sind und von demselben Betätigungsmittel 20 verstellbar sind. Ganz allgemein könnten auch drei, vier oder mehrere Schneidekörper-Paare eingesetzt werden.

Ganz allgemein könnten die Schneidekörper so geschaffen sein, wie es in den Ansprüchen definiert ist.

Zusammenfassend kann festgestellt werden, daß das vorgeschlagene Instrument aus im wesentlichen folgenden Teilen besteht:

Grundkörper 26 und 27, an dem mindestens zwei Schneidekörper angelenkt sind, die durch Verstellen des Körpers bzw. der Klemmkörper sich scherenartig öffnen bzw. schließen. Im Betriebszustand sind die Schneidekanten 24 im wesentlichen außerhalb des Körpers angeordnet, wobei sie den Grundkörper 26 überragen und beim Betätigen des Instrumentes in Zugrichtung mit der Innenwand der Gefäße in Wirkverbindung bringbar sind.

## Patentansprüche

1. Medizinisches Instrument zur Beseitigung von Ablagerungen in Arterien-und oder Venenwänden mit einem in die Gefäße einbringbaren und zumindest teilweise hohlen Körper (10-14) mit einem aus den Gefäßen ausführbaren Zug- und/oder Betätigungsmittel (20), mit in Zugrichtung weisenden und die Ablagerungen beseitigenden Schneidekörpern (21,22) mit Schneidekanten (24) sowie einem in den Körper (10-14) zumindest teilweise hineinragenden Grundkörper (26,27), an dem die in Zugrichtung sich erstreckenden Schneidekörper (21,22) angelenkt sind,
dadurch gekennzeichnet,
daß zumindest zwei als zweiarmige Hebel ausgebildete Schneidekörper (21,22) vorgesehen sind, die zusammen ein Schneidekörperpaar bilden, wobei die Schneidekörper mit einer gemeinsamen Schwenkachse (36) und jeweils einem Kraft- und einem Lastarm (43,44) versehen sind.

2. Instrument nach Anspruch 1,
dadurch gekennzeichnet,
daß der in den Körper (10-14) hineinragende Abschnitt (30) des Grundkörpers (26,27) als ein Rohrabschnitt ausgebildet ist, in dem ein Organ (32,34) zur relativen Verstellung des Körpers (10-14) mit Bezug auf den Schneidekörper (21,22) angeordnet ist .

3. Instrument nach Anspruch 1 oder 2,
dadurch gekennzeichnet,
daß das Organ (32,34) als ein Rohrstück oder Drahtstück ausgebildet ist, das von außen her betätigbar ist.

4. Instrument nach einem der Ansprüche 1 bis 3,
dadurch gekennzeichnet,
daß die Schwenkachse (36) der Schneidekörper (21,22) sich quer zur Zugrichtung des Körpers (10-14) erstreckt .

5. Instrument nach einem der Ansprüche 1 bis 4,
dadurch gekennzeichnet,
daß die Schneidekörper (21,22) in ihrer Ruhestellung vollständig oder annähernd vollständig im Körper (10-14) untergbracht sind.

6. Instrument nach einem der Ansprüche 1 bis 5,
dadurch gekennzeichnet,
daß der Grundkörper (26,27) einen Anschlagabschnitt (38) aufweist, der in Ruhestellung der Schneidekörper (21,22) annähernd abstandsfrei zu einer Stirnseite (40) des Körpers (10-14) angeordnet ist.

7. Instrument nach einem der Ansprüche 1 bis 6,
dadurch gekennzeichnet,
daß der Körper (10-14) im wesentlichen als ein Hohlzylinder ausgebildet ist, der die Schneidekörper (21,22) in ihrer Ruhestellung aufnimmt.

8. Instrument nach einem der Ansprüche 1 bis 7,
dadurch gekennzeichnet,
daß die vom Benutzer abgekehrte Stirnseite (41) des Körpers (10-14) eine in Zugrichtung sich erstreckende Öffnung (42) aufweist.

9. Instrument nach einem der Ansprüche 1 bis 8,
dadurch gekennzeichnet,
daß die vom Benutzer abgekehrte Stirnseite (41) des Körpers (10-14) konvex und kantfrei ist.

10. Instrument nach einem der Ansprüche 1 bis 9,
dadurch gekennzeichnet,
daß die Innenwand des Körpers (10,11,12) Druckkörper (46) aufweist, die in ihrer Betriebsstellung mit dem jeweiligen Kraftarm (43) und in ihrer Ruhestellung mit dem jeweiligen Lastarm (44) der Schneidekörper (21,22) in Druckverbindung stehen.

11. Instrument nach einem der Ansprüche 1 bis 10,
dadurch gekennzeichnet,
daß die Druckkörper (46) als radiale Vorsprünge mit Gleitkurven ausgebildet sind.

12. Instrument nach einem der Ansprüche 1 bis 11,
dadurch gekennzeichnet,
daß das Außenprofil des jeweiligen Schneidekörper-Längsschnittes bogenförmig ist.

13. Instrument nach einem der Ansprüche 1 bis 12,
dadurch gekennzeichnet,
daß das als ein Seil-, Rohr- oder Drahtstück ausgebildete Organ (32,34) Klemmkörper (48,49,50) trägt, die abhängig von der Betriebslage der Schneidekörper (21,22) mit dem Kraft- bzw. Lastarm (43,44) des jeweiligen Schneidekörpers (21,22) zusammenarbeiten.

14. Instrument nach einem der Ansprüche 1 bis 13,
dadurch gekennzeichnet,
daß die Klemmkörper (48,49,50) im axialen Querschnitt keilförmig ausgebildet und mit ihren verjüngten Stirnseiten einander zugekehrt sind.

15. Instrument nach einem der Ansprüche 1 bis 14,
dadurch gekennzeichnet,
daß zwei Klemmkörper (48,49,50) vorgesehen sind, zwischen denen die Schwenkachse der Schneidekörper (21,22) angeordnet ist.

16. Instrument nach einem der Ansprüche 1 bis 15,
dadurch gekennzeichnet,
daß einer der Klemmkörper (50) vom Grundkörper (26) getragen ist und aus einem elastisch verformbaren Werkstoff besteht.

17. Instrument nach einem der Ansprüche 1 bis 16,
dadurch gekennzeichnet,
daß der Körper (10-14) und/oder der Grundkörper (26,27) Querbohrungen (60,61) für Führungsmittel aufweist/aufweisen.

18. Instrument nach einem der Ansprüche 1 bis 17,
dadurch gekennzeichnet,
daß die Schneidekörper (21,22) einen als Kammer ausgebildeten Hohlraum (52) definieren, durch den sich das Organ (35) hindurch erstreckt.

19. Instrument nach einem der Ansprüche 1 bis 18,
dadurch gekennzeichnet,
daß die an die Schneidekanten (24) sich anschließenden Flanken (54,55) der Schneidekörper (21,22) zumindest teilweise mit Schneidabschnitten versehen sind.

20. Instrument nach einem der Ansprüche 1 bis 19,
dadurch gekennzeichnet,
daß mindestens zwei Schneidekörperpaare (21,22) in Serie angeordnet sind.

## Claims

1. Medical instrument for removing deposits in artery and/or vein walls, having a body (10-14) which is at least partially hollow and is insertable into the vessels, a tensioning and/or actuating means (20) which is extractable from the vessels, cutting members (21,22) pointing in the direction of pull and removing the deposits, said cutting members being provided with cutting edges (24), and a basic body member (26 27), which protrudes at least partially into the body (10-14) and on which the culting members (21, 22), extending in the direction of pull, are pivotally mounted, characterised in that at least two cutting members (21, 22) are provided, which are configured as two-armed levers and together form a pair of cutting members, the cutting members being provided with a common pivotal axis (36) and a respective force arm (43) and a respective load arm (44).

2. Instrument according to claim 1, characterised in that the portion (30) of the basic body member (26, 27) protruding into the body (10-14) is configured as a tubular portion, in which a means (32, 34) for the relative displacement of the body (10-14) relative to the cutting members (21, 22) is disposed.

3. Instrument according to claim 1 or 2, characterised in that the means (32, 34) is configured as a piece of tube or piece of wire which is actuatable from externally.

4. Instrument according to one of claims 1 to 3, characterised in that the pivotal axis (36) of the cutting members (21, 22) extends transversely relative to the direction of pull of the body (10-14).

5. Instrument according to one of claims 1 to 4, characterised in that, in their inoperative position, the cutting members (21, 22) are accommodated completely or approximately completely in the body (10-14).

6. Instrument according to one of claims 1 to 5, characterised in that the basic body member (26, 27) has a stop portion (38) which, in the inoperative position of the cutting members (21, 22), is disposed with substantially no spacing from an end face (40) of the body (10-14).

7. Instrument according to one of claims 1 to 6, characterised in that the body (10-14) is substantially configured as a hollow cylinder, which accommodates the cutting members (21, 22) in their inoperative position.

8. Instrument according to one of claims 1 to 7, characterised in that the end face (41) of the body (10-14) remote from the user has an opening (42) extending in the direction of pull.

9. Instrument according to one of claims 1 to 8, characterised in that the end face (41) of the body (10-14) remote from the user is convex and smooth.

10. Instrument according to one of claims 1 to 9, characterised in that the internal wall of the body (10, 11, 12) has pressing members (46) which, in their operative position, are in pressure connection with the respective force arm (43) and, in their inoperative position, are in pressure connection with the respective load arm (44) of the cutting members (21, 22).

11. Instrument according to one of claims 1 to 10, characterised in that the pressing members (46) are configured as radial projection members with sliding curves.

12. Instrument according to one of claims 1 to 11, characterised in that the external profile of the respective cutting member's longitudinal section is arcuate.

13. Instrument according to one of claims 1 to 12, characterised in that the means (32, 34), configured as a cable, piece of tube or piece of wire, supports clamping members (48, 49, 50) which, depending on the operational position of the cutting members (21, 22), co-operate with the force arm and load arm (43, 44) of the respective cutting member (21, 22).

14. Instrument according to one of claims 1 to 13, characterised in that the clamping members (48, 49, 50) have a wedge-shaped axial cross-section and face one another with their tapering end faces.

15. Instrument according to one of claims 1 to 14, characterised in that two clamping members (48, 49, 50) are provided, between which members the pivotal axis of the cutting members (21, 22) is disposed.

16. Instrument according to one of claims 1 to 15, characterised in that one of the clamping members (50) is supported by the basic body member (26) and is formed from a resiliently deformable material.

17. Instrument according to one of claims 1 to 16, characterised in that the body (10-14) and/or the basic body member (26, 27) have/has transverse bores (60, 61) for guide means.

18. Instrument according to one of claims 1 to 17, characterised in that the cutting members (21, 22) define a hollow space (52) configured as a chamber, through which the means (35) extends.

19. Instrument according to one of claims 1 to 18, characterised in that the sides (54, 55) of the cutting members (21, 22) communicating with the cutting edges (24) are provided, at least partially, with cutting portions.

20. Instrument according to one of claims 1 to 19, characterised in that at least two pairs of cutting members (21, 22) are arranged in series.

## Revendications

1. Instrument médical pour l'élimination de dépôts dans les parois d'artères ou de veines avec un corps au moins partiellement creux (10 - 14) pouvant être introduit dans les vaisseaux, avec un moyen de traction et/ou d'actionnement (20) pouvant être extrait des vaisseaux, avec des corps de coupe (21, 22) orientés dans la direction de traction et servant à éliminer les dépôts, corps de coupe qui présentent des arêtes de coupe (23) ainsi qu'avec un corps de base (26, 27) pénétrant tout au moins partiellement dans le corps (10 - 14), corps de base (26, 27) sur lequel sont articulés les corps de coupe (21, 22) orientés dans la direction de traction,
caractérisé en ce qu'au moins deux corps de coupe (21, 22) en forme de leviers à deux bras sont prévus, qui ensemble constituent une paire de corps de coupe, et en ce que les corps de coupe sont dotés d'un axe de pivotement commun (36) et comprennent chacun un bras de force et un bras de charge (43, 44).

2. Instrument suivant la revendication 1,
caractérisé en ce que le tronçon (30) du corps de base (26, 27) qui pénètre dans le corps (10 - 14) a la forme d'un tronçon tubulaire, dans lequel est disposé un organe (32, 34) pour le réglage relatif du corps (10 - 14) par rapport au corps de coupe (21, 22).

3. Instrument suivant la revendication 1 ou 2,
caractérisé en ce que l'organe (32, 34) a la forme d'un tronçon de tube ou de fil métallique, qui peut être actionné depuis l'extérieur.

4. Instrument suivant l'une quelconque des revendications de 1 à 3,
caractérisé en ce que l'axe de pivotement (36) des corps de coupe (21, 22) est orienté transversalement à la direction de traction du corps (10 - 14).

5. Instrument suivant l'une quelconque des revendications de 1 à 4,
caractérisé en ce que dans leur position de repos, les corps de coupe (21, 22) sont entièrement ou sensiblement entièrement logés dans le corps (10 - 14).

6. Instrument suivant l'une quelconque des revendications de 1 à 5,
caractérisé en ce que le corps de base (26, 27) présente un tronçon de butée (38), qui dans la position de repos des corps de coupe (21, 22) est disposé sensiblement non distant d'une face frontale (40) du corps (10 - 14).

7. Instrument suivant l'une quelconque des revendications de 1 à 6,
caractérisé en ce que le corps (10 - 14) a en substance la forme d'un cylindre creux, qui reçoit les corps de coupe (21, 22) dans leur position de repos.

8. Instrument suivant l'une quelconque des revendications de 1 à 7,
caractérisé en ce que la face frontale (41) du corps (10 - 14), face qui n'est pas tournée vers l'utilisateur, présente une ouverture (42) orientée dans la direction de traction.

9. Instrument suivant l'une quelconque des revendications de 1 à 8,
caractérisé en ce que la face frontale (41) du corps (10 - 14) qui n'est pas tournée vers l'utilisateur est convexe et exempte d'arêtes.

10. Instrument suivant l'une quelconque des revendications de 1 à 9,
caractérisé en ce que la paroi intérieure du corps (10, 11, 12) présente des corps de pression (46), qui dans leur position active sont en liaison de pression avec le bras de force (43) correspondant et qui dans leur position de repos sont en liaison avec le bras de charge (44) correspondant des corps de coupe (21, 22).

11. Instrument suivant l'une quelconque des revendications de 1 à 10,
caractérisé en ce que les corps de pression (46) ont la forme de protubérances radiales avec des courbes de glissement.

12. Instrument suivant l'une quelconque des revendications de 1 à 11,
caractérisé en ce que - en coupe longitudinale - le profil extérieur des corps de coupe est de forme arquée.

13. Instrument suivant l'une quelconque des revendications de 1 à 12,
caractérisé en ce que l'organe en forme de tronçon de câble, de tube ou de fil métallique (32, 34) porte des corps de serrage (48, 49, 50) qui en fonction de la position de travail des corps de coupe (21, 22) coopèrent avec le bras de force ou le bras de charge (43, 44) du corps de coupe (21, 22) correspondant.

14. Instrument suivant l'une quelconque des revendications de 1 à 13,
caractérisé en ce que - en coupe axiale - les corps de serrage (48, 49, 50) sont en forme de coin et sont tournés l'un vers l'autre par leurs faces frontales amincies.

15. Instrument suivant l'une quelconque des revendications de 1 à 14,
caractérisé en ce que deux corps de serrage (48, 49, 50) sont prévus, entre lesquels est disposé l'axe de pivotement des corps de coupe (21, 22).

16. Instrument suivant l'une quelconque des revendications de 1 à 15,
caractérisé en ce que l'un des corps de serrage (50) est porté par le corps de base (26) et est constitué par un matérieau pouvant être déformé élastiquement.

17. Instrument suivant l'une quelconque des revendications de 1 à 16,
caractérisé en ce que le corps (10 - 14) et/ou le corps de base (26, 27) présente/présentent des forures transversales (60, 61) pour des moyens de guidage.

18. Instrument suivant l'une quelconque des revendications de 1 à 17,
caractérisé en ce que les corps de coupe (21, 22) définissent une cavité (52) en forme de chambre, au travers laquellle s'étend l'organe (35).

19. Instrument suivant l'une quelconque des revendications de 1 à 18,
caractérisé en ce que les flancs (54, 55) qui se raccordent aux arêtes de coupe (21, 22) présentent tout au moins partiellement des tronçons de coupe.

20. Instrument suivant l'une quelconque des revendications de 1 à 19,
caractérisé en ce qu'au moins deux paires de corps de coupe (21, 22) sont disposées en série.
